# EUROPEAN PATENT SPECIFICATION

(11) **EP 2 809 792 B1**
(45) Date of publication and mention of the grant of the patent: **06.05.2020**
(21) Application number: 13724364.8
(22) Date of filing: 24.01.2013
(51) Int. Cl.: C12P 19/12, C12P 19/14, C12P 19/16, C12P 19/22, C13K 7/00

(54) **PROCESS FOR PRODUCING MALTOSE FROM STARCH**
VERFAHREN ZUR HERSTELLUNG VON MALTOSE AUS STÄRKE
PROCÉDÉ DE PRODUCTION DE MALTOSE À PARTIR D'AMIDON

(30) Priority: 31.01.2012 EP 12000621; 29.02.2012 EP 12001375
(43) Date of publication of application: 10.12.2014
(73) Proprietor: Cargill, Incorporated, Wayzata, MN 55391 (US)
(72) Inventor: FURLAN, Tiziano, I-45100 Rovigo (IT); NATALONI, Luigi, I-40139 Bologna (IT); TOLOMELLI, Patrizia, I-44124 Ferrara (IT)
(74) Representative: Jantschy, Jasmin
(86) International application number: PCT/IB2013/000864
(87) International publication number: WO 2013/114222

(56) References cited:
- DE-A1- 1 935 330
- US-A- 5 198 120
- US-B1- 6 346 400
- DATABASE WPI Week 200257 Thomson Scientific, London, GB; AN 2002-531597 XP002681976, -& JP 2002 101896 A (NIPPON SHIRYO KOGYO KK) 9 April 2002 (2002-04-09)

## Description

### Field of the Invention

The present invention relates to a process for producing a syrup which is rich in maltose.

### Background of the Invention

Methods allowing the production of maltose rich syrups are already well-known.

US 5,141,859 allows high-purity maltose to be manufactured through the steps of liquefaction of starch, saccharification of the liquefied substance with general purpose enzymes further saccharification with an enzyme which hydrolyzes oligosaccharides of trisaccharide or more. The liquefact is saccharified by using at least two enzymes selected from the group consisting of beta-amylase, iso-amylase and pullulanase. In the next step, maltogenic alpha-amylase or gluco-amylase is applied. The saccharification process can be executed setting the standard of the period until the purity of maltose reaches equilibrium, normally 24 to 72 hours.

US 6,284,498 relates to a method of manufacturing a maltose-rich syrup comprising the successive steps consisting of carrying out a liquefaction of a starch milk, carrying out a saccharification of the liquefied starch milk in the presence of a maltogenic alpha-amylase, continuing the saccharification of the liquefied starch milk in the presence of a beta-amylase and at least one debranching enzyme chosen from the group consisting of pullulanase and iso-amylases with a view to obtaining a syrup which is rich in maltose. This method allows obtaining syrups having a richness of 86% maltose and containing 6.6% glucose and 7.4% DP3+.

US 6,346,400 relates to a process for the preparation of a maltose rich syrup comprising of liquefaction, saccharification in the presence of beta-amylase and at least one debranching enzyme selected from the group of pullulanases and iso-amylases, carrying out molecular sieving of the liquefied starch milk so as to collect a fraction enriched with maltose and bringing said fraction enriched in maltose into contact with a maltogenic alpha-amylase with a view to obtaining a maltose rich syrup.

DE 19 35 330 A1 discloses a method for the production of a maltose liquid. Here, the alpha-amylase used in the liquefaction is inactivated before the saccharification and then the saccharification is carried out in the presence of beta-amylase and the debranching enzyme pullulanase for obtaining a maltose containing syrup comprising at least 85% maltose based on dry matter and less than 1.5% glucose based on dry matter.

JP 2002 101896 A discloses a method for the production of a maltose liquid.

There is still a further need of having a process providing a syrup rich in maltose and low in DP1 and preferably further low in DP3.

### Summary of the Invention

The current invention relates to a process for preparing a maltose containing syrup comprising the successive steps of
a) Carrying out liquefaction of a starch milk,
b) Carrying out saccharification of the liquefied starch milk in the presence of alpha-amylase, and beta-amylase and a debranching enzyme selected from the group of pullulanase, iso-amylase and mixtures thereof,
c) Further adding maltogenic alpha-amylase and/or iso-amylase, and
wherein in step b) the saccharification is taking place in presence of from 1% to 4% of residual activity of total amount of alpha-amylase applied in the liquefaction of step a);
and wherein after step c) additional alpha-amylase is added at about 70 to 85% spent time of total saccharification time, for obtaining a maltose containing syrup comprising at least 85% maltose based on dry matter and less than 1.5% glucose based on dry matter.

### Detailed Description

The current invention relates to a process for preparing a maltose containing syrup comprising the successive steps of
a) Carrying out liquefaction of a starch milk,
b) Carrying out saccharification of the liquefied starch milk in the presence of alpha-amylase, and beta-amylase and a debranching enzyme selected from the group of pullulanase, iso-amylase and mixtures thereof,
c) Further adding maltogenic alpha-amylase and/or iso-amylase, and
wherein in step b) the saccharification is taking place in presence of from 1% to 4% of residual activity of total amount of alpha-amylase applied in the liquefaction of step a); and wherein after step c) additional alpha-amylase is added at about 70 to 85% spent time of total saccharification time, for obtaining a maltose containing syrup comprising at least 85% maltose based on dry matter and less than 1.5% glucose based on dry matter.

The liquefaction is carried out in presence of alpha-amylase.

The liquefaction and saccharification of starch can be conducted in various ways, but the current invention demonstrates that combining the liquefaction with a specific saccharification step allows obtaining a maltose syrup comprising at least 85% maltose (= DP2), at least 87%, at least 90% maltose based on dry matter and less than 1.5% glucose (= DP1) based on dry matter, preferably less than 1% glucose based on dry matter, and preferably comprising less than 10% DP3, more preferably comprising less than 10% of oligosaccharides having a polymerisation degree of 3 or more (= DP3+).

The liquefaction is conducted on starch of any botanical origin. For instance it may originate from wheat, corn or potato.

The liquefaction is to be considered as a controlled hydrolysis of starch milk, in the presence of enzymes such as alpha-amylase, and so as to obtain a liquefied starch milk with a low degree of conversion. Thus the conditions of temperature, pH, enzyme (type as well as concentration) are selected in such a way that they make it possible to obtain a DE (= dextrose equivalent) of not more than 6, preferably from 4 to 5.

Preferably the liquefaction is carried out in three steps, the first step is consisting in heating the starch milk at a temperature in the range of 105 to 108°C and in presence of a thermostable alpha-amylase for a few minutes, typically from 8 to 15 minutes, not longer than 20 minutes. The second step is consisting of heating the starch milk thus treated at a temperature in the range of 140 to 160°C, preferably in the range of 145-155°C for a few minutes, for a time period of 5 to 8 minutes, but not longer than 20 minutes. After cooling down to about 95 to 100°C, a second small dosage of alpha-amylase is added and the liquefaction continued for another 30 to 50 minutes and is thus tuned so to achieve a starch slurry with a D.E. of 4 to 6, preferably from 4 to 5.

The liquefaction according to the current invention allows preparing a D.E. of 4 to 6, preferably from 4 to 5, wherein the composition of the oligosaccharides (DPn) is pre-fine-tuned for the subsequent saccharification.

Once the liquefaction step is ended, a controlled inhibition is conducted such that only a partial inhibition of the alpha-amylase is carried out. Preferably the partial inhibition is conducted at a pH of 3.5 to 4 at a temperature not higher than 100°C. The partial inhibition is conducted during a time period of 1 to 10 minutes. The residual (remaining active) alpha-amylase is further used in the subsequent saccharification step. Preferably, the residual alpha-amylase corresponds to from 5 to 15% of the total amount added in the second dosing of the liquefaction. Finally, the residual alpha-amylase corresponds to 7% to 12% of the total amount added in the second dosing of the liquefaction. Compared with the actual total amount of alpha-amylase added during the liquefaction (= dose 1 + second dosage) it corresponds to 1% to 4%, preferably from 1.4% to 3% of residual activity of total amount of alpha-amylase.

Preferably the saccharification of liquefied starch milk is carried out in presence of alpha-amylase, and beta-amylase and as debranching enzyme, pullulanase, wherein the saccharification is taking place in presence of residual amount of alpha-amylase applied in the liquefaction of step a), in presence of from 1% to 4%, or in presence of 1.4% to 3% of residual activity of total amount of alpha-amylase applied in the liquefaction.

Saccharification is then continued by adding a beta-amylase and a debranching enzyme selected from the group of pullulanase, iso-amylase and mixtures thereof. Preferably pullulanase is added. The addition of debranching enzyme makes it possible to hydrolyse the 1,6- linkages and thus to reduce the quantity of highly branched oligosaccharides. Preferably the ratio of beta-amylase to debranching enzyme is from 1:1 to 1:4. Preferably the ratio of beta-amylase to pullulanase is from 1:1 to 1:4. Ratios from 1:1 to 1:5 or even up to 1:10 are part of the invention. Preferably, in applying pullulanase as debranching enzyme, the ratio of beta-amylase to pullulanase is from 1:2 to 1:4 and preferably the higher upper-end from 1:3 to 1:4 is applied.

Maltogenic alpha-amylase and/or iso-amylase is added to the so far treated starch milk, at about 20 to 50% spent time of the total saccharification time, preferably at about 25 to 35%, preferably at about 25% to 30% spent time of the total saccharification time. The maltogenic alpha-amylase is an exo-acting alpha-amylase which is responsible for the exo-hydrolysis of 1,4-alpha-glucosidic linkages. Iso-amylase is a debranching enzyme which is hydrolysing the 1,6-linkages and reduces the amount of the reversion products.
In a typical process the total saccharification time is about 16 to 30 hours, preferably 20 to 24 hours and the maltogenic alpha-amylase and/or iso-amylase is added after 7 to 8 hours of the total saccharification time are lapsed.
The saccharification is thus continued until a maltose rich syrup is obtained which is containing at least 85%, or at least 87%, at least 90% maltose based on dry matter and less than 1.5% glucose based on dry matter, preferably less than 1% glucose based on dry matter.

The saccharification is conducted such that a syrup rich in maltose is obtained such that it contains at least 85% maltose based on dry matter and less than 1.5% glucose based on dry matter, preferably less than 1% glucose based on dry matter and less than 10% of DP3 or less than 10% of polymers having a degree of polymerisation of 3 or more (= DP3+) based on dry matter, preferably less than 5% DP3+. Even more preferably the polymers having a degree of polymerisation higher than 3 are negligible and the amount of polymers having a degree of polymerisation of 3 is below 5%, more preferably below 3%, most preferably below 1% based on dry matter of the syrup.

Finally more towards the end of the saccharification step, additional alpha-amylase is added. This specific low amount may further improve the subsequent down-streaming process. The alpha-amylase is added at about 70 to 85% spent time of total saccharification time, preferably at about 80 to 83% spent time of the total saccharification time. This can correspond to about 4 hours before ending the saccharification process.

The process of the current invention allows to obtain product with very high content (= at least 85%, or at least 87%, at least 90%) of maltose while the content of glucose is below 1.5%, with low DP3 amount and wherein the presence of long chain oligosaccharides is reduced. The composition of DPn is different from the composition that is usually obtained after liquefaction and saccharification. In particular the use of residual alpha -amylase in the subsequent saccharification step and the further addition of alpha-amylase towards the end of the saccharification contributes to the change of the composition of the DPn (oligosaccharide) fraction. The amount of higher oligosaccharides (= longer chains) is reduced.

The thus obtained saccharified syrup can be purified according to the well-known demineralization processes such as by applying ion exchange resins. Alternatively, the saccharified syrup may be filtered on a precoat filter or by microfiltration on membranes and then followed by demineralization.

Further disclosed is a maltose containing syrup comprising at least 85% maltose based on dry matter, and less than 1.5% glucose based on dry matter, for example less than 1% glucose based on dry matter and having a dry matter content of 25-75%, for example 30 to 65%, or 35 to 60% and said maltose containing syrup being obtained by the process of the current invention. Surprisingly it was found that by applying the process of the current invention, directly without further purification steps, the maltose syrup with the current composition on dry matter was obtained.

For example, the syrup is containing at least 87% maltose, for example, at least 89%, at least 90% maltose based on dry matter and less than 1% of glucose. Finally the syrup is containing less than 10% of DP3, or for example less than 10% of polymers having a polymerisation degree higher than 3, based upon dry matter of the syrup, for example less than 8% of DP3+ (= oligosaccharides of polymerisation degree of 3 and more), even less than 5% DP3+. This syrup is having a dry matter content of 25-75%, for example 30 to 65%, or 35 to 60%.

So far high maltose (up to 80%) syrups with low amount of glucose have been obtained, as well as very high maltose (up to 90%) with significant residual glucose (5 to 7%). The current invention has demonstrated that by applying the liquefaction according to the current process and combining it with the saccharification step as is claimed in the current invention, surprisingly, it is feasible to obtain maltose syrups with very high content of maltose (at least 85%) and low amounts of glucose (less than 1.5%). And finally also the content of DP3 is low, less than 10%, preferably less than 5%. Furthermore the DPn fraction starting with DP4 has a significant different composition so that the amount of long chain oligosaccharides is reduced. This changed composition makes the final product of the current invention more stable and it is a better precursor for maltitol production through hydrogenation.

This maltose rich syrup obtainable through the process of the current invention can be used in food applications or industrial applications, or can be used as precursor for maltitol or as a precursor of crystalline maltose or as feed material in molecular sieving.

As precursor for maltitol, the demineralized saccharified syrup can be directly hydrogenated for obtaining a maltitol rich syrup.

Furthermore the maltose rich syrup can be applied as feed substrate in a molecular sieving or it can directly be crystallized for obtaining crystalline maltose.

The invention will hereunder be illustrated in the form of the following examples.

### Examples

### Example 1

### Liquefaction

Starch slurry at dry matter content between 27-35% ds (is dry matter) was liquefied, after pH adjustment at 5,8(±1) and after dosage of 0,08-0,1% of alpha-amylase (Spezyme (Genencor)) by using jet cooker at 108°C. After 8-15 minutes, the pasting temperature was reduced to 100°C by atmospheric flash and then the slurry was sent to the second jet at 152°C. After 5-8 minutes of pasting, the slurry was cooled down to 100°C and a second dosage (0,025%) of the same alpha-amylase was added and this amount is tuned in order to reach 4-6 DE (target 4,5).

After 30-50 minutes of reaction on the agitated column at 100°C, the pH of the liquefact was adjusted at 3-4 (target 3.5-4) at 100°C for max 10 minutes to inhibit part of the alpha-amylase. After this treatment, 7 to 10% of the alpha-amylase added as second dosage was maintained.

### Example 2

### Saccharification - RECIPE 1

The product of example 1 was used. Saccharification started at pH 4,8-5,0 in presence of residual alpha-amylase and 0,1% of beta-amylase (Optimalt BBA (Genencor)) and 0,4% of pullulanase (Promozyme D2 (Novozyme)). After 7-8h reaction 0,02% of maltogenic alpha-amylase (Maltogenase (Novozyme)) was added.
At least 4 hours before unloading the saccharificator, 0,1-0,2% of alpha-amylase (Liquozyme X (Novozyme)) was added. After a total saccharification time of 24-30h the following composition was reached: glucose <1%, maltose (= DP2) 85-87%, DP3 (= oligosaccharide with polymerization degree of 3) 7-10%, DP4+ (oligosaccharides with polymerization degree of 4 and more) < 5%.

Purification is carried out as the purification for regular glucose syrups.

### Example 3

### Saccharification - RECIPE 2

The product of example 1 was used. Saccharification started at pH 4,8-5,0 in presence of residual alpha-amylase and 0,1 % of beta-amylase (Optimalt BBA (Genencor)) and 0,4% of pullulanase (Promozyme D2 (Novozyme)). and 0,1% of iso-amylase . After 7-8h reaction 0,1% maltogenic alpha-amylase (Maltogenase (Novozyme)) was added.

At least 4 hours before unloading the saccharificator, 0,1-0,2% of alpha-amylase (Liquozyme X (Novozyme)) was added. After a total saccharification time of 24-30h the following composition was reached: glucose <1%, maltose (=DP2) 87-90%, and DP3 is 4 to 6%.

## Claims

1. A process for preparing a maltose containing syrup comprising the successive steps of
a) Carrying out liquefaction of a starch milk
b) Carrying out saccharification of the liquefied starch milk in the presence of alpha-amylase, and beta-amylase and a debranching enzyme selected from the group of pullulanase, iso-amylase and mixtures thereof,
c) Further adding maltogenic alpha-amylase and/or iso-amylase,
and
wherein in step b) the saccharification is taking place in presence of from 1% to 4% of residual activity of total amount of alpha-amylase applied in the liquefaction of step a);
and wherein after step c) additional alpha-amylase is added at about 70 to 85% spent time of total saccharification time, for obtaining a maltose containing syrup comprising at least 85% maltose based on dry matter and less than 1.5% glucose based on dry matter.

2. The process according to claim 1 wherein in step b) the ratio of beta-amylase to debranching enzyme is from 1:1 to 1:4.

3. The process according to claim 1 or 2 wherein in step b) the debranching enzyme is pullulnase.

4. The process according to any one of claim 1 to 3 wherein in step a) the liquefaction is taking place until a DE of not more than 6.

5. The process according to any one of claim 1 to 4 wherein in step c) the addition of maltogenic alpha-amylase and/or iso-amylase is taking place at about 20 to 50% spent time of the total saccharification time.

6. The process according to anyone of claim 1 to 5 wherein in step c) the maltose containing syrup is comprising at least 85% maltose based on dry matter and less than 1.5% glucose based on dry matter and less than 10% DP3 based on dry matter.

## Patentansprüche

1. Verfahren zur Herstellung eines maltosehaltigen Sirups, umfassend die aufeinanderfolgenden Schritte von
a) Durchführung der Verflüssigung einer Stärkemilch
b) Durchführung der Verzuckerung der verflüssigten Stärkemilch in Gegenwart von Alpha-Amylase und Beta-Amylase und eines Debranching-Enzyms, ausgewählt aus der Gruppe der Pullulanase, Iso-Amylase und Mischungen davon,
c) Weiterer Zugabe von maltogener Alpha-Amylase und/oder Iso-Amylase, und
wobei in Schritt b) die Verzuckerung in Gegenwart von 1% bis 4% der Restaktivität der Gesamtmenge an Alpha-Amylase, die bei der Verflüssigung von Schritt a) eingesetzt wird, stattfindet);
und wobei nach Schritt c) zusätzliche Alpha-Amylase bei etwa 70 bis 85% verbrachter Zeit der gesamten Verzuckerungszeit zugegeben wird, um einen maltosehaltigen Sirup zu erhalten, der mindestens 85% Maltose auf Trockenmassebasis und weniger als 1,5% Glucose auf Trockenmassebasis umfasst.

2. Verfahren nach Anspruch 1, wobei in Schritt b) das Verhältnis von Beta-Amylase zu Debranching-Enzym von 1:1 bis 1:4 beträgt.

3. Verfahren nach Anspruch 1 oder 2, wobei in Schritt b) das Debranching-Enzym Pullulanase ist.

4. Verfahren nach einem der Ansprüche 1 bis 3, wobei in Schritt a) die Verflüssigung bis zu einem D.E. von nicht mehr als 6 stattfindet.

5. Verfahren nach einem der Ansprüche 1 bis 4, wobei in Schritt c) die Zugabe von maltogener Alpha-Amylase und/oder Iso-Amylase bei etwa 20 bis 50% verbrachter Zeit der gesamten aufgewendeten Verzuckerungszeit erfolgt.

6. Verfahren nach einem der Ansprüche 1 bis 5, wobei in Schritt c) der maltosehaltige Sirup mindestens 85% Maltose bezogen auf die Trockenmasse und weniger als 1,5% Glucose bezogen auf die Trockenmasse und weniger als 10% DP3 bezogen auf die Trockenmasse umfasst.

## Revendications

1. Procédé de préparation d'un sirop contenant du maltose comprenant les étapes successives consistant à
a) effectuer une liquéfaction d'un lait d'amidon
b) réaliser une saccharification du lait d'amidon liquéfié en présence d'alpha-amylase, de bêta-amylase et d'une enzyme de déramification choisie parmi le groupe constitué de pullulanase, iso-amylase et leurs mélanges,
c) ajouter de plus une alpha-amylase maltogénique et/ou iso-amylase,
et
dans lequel, à l'étape b), la saccharification a lieu en présence de 1 % à 4 % d'activité résiduelle de la quantité totale d'alpha-amylase appliquée lors de l'étape de liquéfaction a) ;
et dans lequel, après l'étape c), une alpha-amylase supplémentaire est ajoutée entre environ 70 et 85% de temps écoulé du temps total de saccharification, pour obtenir un sirop contenant du maltose comprenant au moins 85 % de maltose sur la base de la matière sèche et moins de 1,5 % de glucose sur la base de la matière sèche.

2. Procédé selon la revendication 1, dans lequel, à l'étape b), le rapport entre la bêta-amylase et l'enzyme de déramification est compris entre 1:1 et 1:4.

3. Procédé selon la revendication 1 ou 2, dans lequel, à l'étape b), l'enzyme de déramification est une pullulanase.

4. Procédé selon l'une quelconque des revendications 1 à 3, dans lequel, à l'étape a), la liquéfaction a lieu jusqu'à un DE de pas plus de 6.

5. Procédé selon l'une quelconque des revendications 1 à 4, dans lequel, à l'étape c), l'ajout d'alpha-amylase et/ou d'iso-amylase maltogéniques a lieu entre environ 20 à 50 % de temps écoulé du temps de saccharification total.

6. Procédé selon l'une quelconque des revendications 1 à 5, dans lequel, à l'étape c), le sirop contenant du maltose comprend au moins 85 % de maltose sur la base de la matière sèche et moins de 1,5 % de glucose sur la base de la matière sèche et moins de 10 % de DP3 sur la base de la matière sèche.
